(19) Europäisches Patentamt
European Patent Office
Office européen des brevets

(11) **EP 1 958 963 B1**

(12) **EUROPEAN PATENT SPECIFICATION**

(45) Date of publication and mention
of the grant of the patent:
**05.01.2011 Bulletin 2011/01**

(51) Int Cl.:
*C07K 14/805* (2006.01)    *A61K 38/42* (2006.01)
*A61K 9/127* (2006.01)    *A61K 31/198* (2006.01)

(21) Application number: **08151385.5**

(22) Date of filing: **13.02.2008**

(54) **Oxygen-carrying blood substitute preparations**

Sauerstoffhaltige Blutersatzpräparate

Préparation de substitute de sang porteuse d'oxygène

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.02.2007 JP 2007032978**

(43) Date of publication of application:
**20.08.2008 Bulletin 2008/34**

(73) Proprietor: **NIPRO CORPORATION**
**Osaka-shi, Osaka 531-8510 (JP)**

(72) Inventors:
• **Kai, Toshiya**
  **Kusatsu Shiga (JP)**
• **Nishida, Seiji**
  **Kusatsu Shiga (JP)**
• **Katayama, Naohisa**
  **Kusatsu Shiga (JP)**
• **Hori, Nobuyuki**
  **Yokohama Kanagawa (JP)**
• **Manabe, Yoshihisa**
  **Nara Nara (JP)**
• **Suka, Yuko**
  **Yokohama Kanagawa (JP)**

(74) Representative: **Reitstötter - Kinzebach**
**Patentanwälte**
**Sternwartstrasse 4**
**81679 München (DE)**

(56) References cited:
**EP-A- 1 466 649    EP-A- 1 714 691**
**WO-A-01/01775    WO-A-92/02239**

## Description

Field of the Invention

This invention relates to an oxygen-carrying blood substitute preparation containing an oxygen-carrying substance capable of reversibly binding and releasing oxygen in vivo. The preparation finds use in the medical field, for example, as a blood substitute to be transfused to a hypovolemic patient following large volume hemorrhage, for supplying oxygen to ischemic sites and tumor tissues, as a perfusion solution for preserving native organs, and as a medium for culturing cells.

Background Art

It is desirable for oxygen-carrying blood substitute preparation such hemoglobin-based compositions or porphyrin-metal complex-based compositions to contain an amount of methemoglobin or an equivalent thereof as low as possible. The main cause of an elevated level of methemoglobin and an equivalent thereof is autooxidation during the production process and/or storage period. Attempts have been made, therefore, to remove oxygen from hemoglobin solutions by deoxygenating the solution with an inert gas during the production process. See, Japanese Patent Nos. 2,709,419 and 3,466,516.

However, the above procedure has been proven to be not fully effective when used alone because hemoglobin is highly susceptible to autooxidation. Consequently, it is necessary to decrease the oxygen concentration as low as possible in the atmosphere in which the hemoglobin-based preparation is packaged in a container or bag. This necessarily increases the production costs.

Summary of the Invention

It is, therefore, an object of the present disclosure comprising the present invention to provide an oxygen-carrying blood substitute preparation according to claim 1 which is stable during storage so that the amount of methemoglobin or an equivalent thereof remains at a low level during the storage. In another instance of the present disclosure, an oxygen-carrying blood substitute preparation without increasing the production costs is provided.

As a result of vigorous studies, we have found that the above problems may be solved by incorporating into an oxygen-carrying blood substitute preparation an amount of a reducing agent which has a low toxicity and thus tolerable as an additive for pharmaceutical preparations, typically cysteine. The present invention has its basis on the above finding. Furthermore, we have found that a storage-stable preparation may be provided by filling an inner bag with the above oxygen-carrying preparation, and then placing the inner bag in an outer envelope together with an oxygen absorbing packet.

Effects of the Invention

The preparation containing an oxygen-carrying substance according to the present invention is stable and maintains a low content of methemoglobin or an equivalent thereof. The preparation of the present invention may be packaged in a flexible bag in an atmosphere having an oxygen concentration as high as atmospheric air. This assists prevention of cost increase.

The oxygen-carrying blood substitute preparation of the present invention comprises an oxygen-carrying substance and a reducing agent.

A. Oxygen-carrying substance

The oxygen-carrying substance may be any substance known to be capable of reversibly binding and releasing oxygen in vivo including substances of native origin such as those derived from red blood cells and selected from a liposome-encapsulated hemoglobin, a conjugate of porphyrin-metal complex complex and albumin, a conjugate of polyethylene glycol (PEG)-modified albumin and porphyrin-metal complex conjugate, hemoglobin, intra- and intermolecularly crosslinked hemoglobin, a hemoglobin polymer, a PEG-modified hemoglobin polymer.

Liposome-encapsulated hemoglobin

"Liposome-encapsultated hemoglobin" (HbV) refers to liposomes containing hemoglobin in the inner space therein. Liposomes normally refer to closed vesicles comprising outer lamellar lipid phase and inner aqueous phase. The liposome structure may be multilamelar or unilamelar. The liposomes preferably have a volume average particle size from 10nm to 500nm, preperably from 30nm to 500nm. The volume average particle size may be determined, for example, by the dynamic light scattering method.

Liposomes may be produced from any lipid known in the art. See, Liposome Technology, 2nd edition, Vol. 1, 41(1993). Non-limiting examples thereof include glycolipids, sterols, fatty acids, phospholipids, amphiphilic alkyl amino acid derivatives, dialkyl dimethylammonium compounds, polyglycerol alkyl ethers, polyoxyethylene alkyl ethers, alkylglycosides, alkylmethylglucamide, sucrose fatty acid esters, amphiphilic polyoxyethylene-polylactic acid block copolymers (WO93/00101), long-chain alkylamines such as tetradecylamine, hexadecylamine and stearylamine, long-chain fatty acid hydrazides such as myristoyl hydrozide, palmitoyl hydrazide and stearoyl hydrazide, and a mixture of these lipids.

Particularly preferred liposomes are produced from polyethylene glycol-non-phosphorylated lipid complexes having a polyethylene glycol content from 0.1 to 30 mole % based on the total lipid components.

Glycolipids include both glycerolipids and sphingolipids. Examples of glycerolipids include digalactosyl glycerides such as digalactosyl dilauroylglyceride, digalactosyl dimyristoylglyceride, digalactosyl dipalmitoylglyceride or digalactosyl distearoylglyceride; and galactosyl glycerides such as galactosyl dilauroylglyceride, galactosyl dimyristoylglyceride, galactosysl dipalmitoylglyceride and galactosyl distearoylglyceride.

Examples of sterols include cholesterol, cholesterol hemisuccinate, $3\beta$-[N-(N'-N'-dimethylaminoethyl)carbamoyl] cholesterol, ergosterol and lanosterol.

Examples of phospholipids include phosphatidylcholine, phosphatidylethanolamine, phosphatidylserine, phosphatidylglycerol, phosphatidylinositol, phosphatidylic acid, lizophosphatidylcholine, sphingomyelin, yolk lecithin, soybean lecithin or hydrogenated phospholipids.

Porphyrin metal complex-albumin conjugate

Porphyrin metal complex refers to a coordination compound having a porphin ring and a metal atom coordinated in the center of the porphin ring. For use as an oxygen-carrying component, the complex must be capable of reversibly binding oxygen. Preferable metal species include iron, cobalt and chromium. Fe(II) and Co(II) are particularly preferred. Specific examples of the porphyrin complexes include iron (II) complexes of tetraphenylporphyrin, protoporphyrin, octaalkylporphyrin and derivatives thereof. The porphyrin metal complex may be modified with polyethylene glycol (PEG) having a molecular weight of 200-4,000,000, preferably 1,000-50,000.

The albumin components forming a conjugate with the porphyrin complex include human serum albumin, animal serum albumin, recombinant human albumin (rHSA), recombinant animal serum albumin, and polymers thereof. rHSA is particularly preferred for the prevention of microorganism infection.

One specific example of the porphyrin complex-albumin conjugate is 2-[8-{N-(2-methylimidazolyl)}octanoyloxymethyl]-5,10, 15,20-tetrakis($\alpha,\alpha,\alpha, \alpha$-0-pivaloylamino) phenylporphinato-iron(II) entrapped in the hydrophobic pockets of recombinant albumin. See, Tsuchida, E. et al., Bioconjugate Chemistry, 8, 534-538, 1997.

Hemoglobin

As a hemoglobin component, native hemoglobin from various origins or recombinant hemoglobin may be used. Recombinant hemoglobin is preferable. Also included in usable hemoglobin component are intra- or intermolecularly crosslinked hemoglobins, polymers such as dimer of hemoglobin, and PEG modified hemoglobin polymers. Specific examples thereof include intramolecularly crosslinked hemoglobin with glutaraldehyde, intermolecularly crosslinked hemoglobin polymers produced via disulfide linkage exchange reactions using carbodiimide or N-succinimidyl-3-(2-pyridylthio)propionate (SPDP), intermolecularly crosslinked PEG-modified hemoglobin produced via disulfide linkage exchange reactions using PEG maleimide, and intermolecularly crosslinked PEG-modified hemoglobin polymers produced via disulfide linkage exchange reactions of the above intermolecularly crosslinked hemoglobin polymers using PEG maleimide.

Oxygen-carrying liposomes may be prepared from a mixture of a lipid component and a hemoglobin component by any method known in the art including the reverse evaporation method, the high pressure extrusion method or the microfluidizing method. It is desirable to prepare the liposome-encapsulated hemoglobin preparation at a temperature below 10°C in order to prevent hemoglobin from denaturing.

Liposomes may be prepared from PEG-lipid conjugates. Normally, the linkage between PEG and lipid utilizes a spacer molecule. The spacer molecule must possess at least a functional group, capable of binding PEG and another functional group capable of binding the lipid. Typical examples are succinic acid and amino acids. Trifunctional amino acids having two functional groups at the both terminals and a functional group between the both terminals. Examples thereof include lysine, asparagine, glutamine, aspartic acid, glutamic acid, serine, threonine and tyrosine. Trifunctional amino acids having the same functional groups at the both terminals and a second functional group between them are particularly preferable including amino acids having a terminal carboxylic group and two amino groups such as lysine, asparagine or glutamine, and amino acids having two terminal carboxylic groups and an amino group such as aspartic acid or glutamic acid.

Porphyrin metal complex-albumin conjugates may be produced following the production method of albumin-heme conjugate reported by E. Tsuchida et al. in Bioconjugate Chemistry, 8, 534-538, 1997.

Hemoglobin may be prepared from lysate of human red blood cells by removing stroma. The oxygen-carrying preparation of the present invention is prepared from a stroma-free hemoglobin solution having a pH between 7.0 and 7.5. The pH may be adjusted with an aqueous solution of sodium hydroxide, sodium carbonate or sodium hydrogen carbonate. Alternativly, buffer solutions such as Tris, bis-Tris, HEPES or phosphate buffer solutions may be used for adjusting the pH of the hemoglobin solution.

B. Reducing agent

The reducing agent used in the present invention to L-cysteine. The reducing agent is capable of preventing autooxidation of hemoglobin to methemoglobin in the presence of oxygen, and is physiologically acceptable at a concentration of use.

The reducing agent is added to an oxygen-carrying substance in the form of a solution or dispersion in injectable water, physiological saline or a buffer solution such as PBS to facilitate mixing with the oxygen-carrying substance. The concentration of the reducing agent in the mixture ranges from 0.5 to 10 mM,

C. Dual package and preparation of oxygen-carrying blood substitute preparation

The oxygen-carrying substance and the reducing agent are thoroughly deoxygenated by purging with an inert gas before mixing. Then the mixture is packaged by filling or a inner bag with the mixture under sterile conditions in an oxygen-deficient atmosphere. The inner bag is made from an oxygen-impermeable material such as polyvinylidene chloride or ethylene-vinyl alcohol copolymers. In one istance the inner bag may also be made from a laminate of a polyethylene film and aluminum foil.

The inner bag filled with the oxygen-carrying preparation of the present invention is packaged together with an oxygen-absorbing packet in an outer envelope also made of an oxygen-impermeable material. The oxygen-absorbing material may be ascorbic acid based or iron based material. The amount thereof to be placed in the space between the inner bag and the outer envelope may vary depending upon the volume of said space and is sufficient to decrease the oxygen level in the space to less than 0.1 %. For example, an amount of the oxygen absorbing material capable of absorbing a volume of oxygen as much as 5 to 10 times of the volume of said space may be used. The dual packaging as above contributes to the storage stability of the oxygen-carrying preparation by essentially all oxygen remaining in the space between the inner bag and the outer envelope, and the oxygen-carrying preparation in the inner bag is protected from exterior oxygen by dual barriers.

The packaging operation of oxygen-carrying preparations in general must be performed in a low-oxygen concentration embironment. However, the oxygen-carrying preparation of the present invention may be packaged in an atmosphere having much higher oxygen concentrations. For example, the packaging operation may be performed in an atmosphere having an oxygen concentration as high as the atmospheric air. As demonstrated in the examples below, the preparation is stable at least for one month even when packaged in an atmosphere having an oxygen concentration as high as the atmospheric air. Accordingly, an equipment for creating a low oxygen environment is not required and thus the increase in the production costs is prevented.

In one instance of the present disclosure, the oxygen-carrying preparation may further contain a variety of pharmaceutically acceptable additives which are known and conventional in the art. The oxygen-carrying preparation of the present invention may be stored at a temperature from -20°C to 60 °C, preferably from 4°C to 25°C.

Examples

The present invention will be described in detail making reference to the following examples. In these examples, a liposome-encapsulated hemoglobin L-cysteine are used as an oxygen-carrying substance.

Part I. Method for determining percent methemoglobin (%Met)

1. Preparation of various solutions

A solution containing Hb at a concention of 5g/dL is prepared in a phosphate buffer (PBS), pH 7. An aqueous solution containing 5g/dL of $NaNO_2$ and an aqueous solution containing 10g/dL of L-Cys are also prepared. The L-Cys solution is deoxygenated by bubbling with nitrogen gas in a glass vial and stored under anaerobic conditions until use.

2. Preparation of standard absorption curves at 0% Met and 100% Met.

a) Methemoglobin (MetHb)

To 1mL of the above Hb solution is added 50$\mu$L of the above NaNO$_2$ solution and allowed to stand at room temperature for 1 hour. To a necked photometric cuvette containing 5mL of PBS are added 10$\mu$L of the above Hb solution an.d then additional 150$\mu$L of PBS. The absorbance of the MetHb solution is measured over a wavelength range from 300nm to 500nm and recorded as an absorption curve.

b) Deoxyhemoglobin (DeoxyHb)

To 1mL of the above Hb solution is added 50$\mu$L of PBS. A necked photometric cuvette containing 5mL of PBS is deoxygenated by bubbling with nitrogen gas for 10 minutes. Then 150$\mu$L of the above L-Cys solution and 10$\mu$L of the above Hb solution are added to the cuvette. The absorbance of the Hb solution is measured over a wavelength range from 300nm to 500nm and recorded as an absorption curve.

3. Calculation of absorbance at wavelength of 405nm (A405) and absorbance at wavelength of 430nm (A430)

The absorption curves for MetHb and DeoxyHb are overlaid to each other. A baseline is drawn on the overlaid curves connecting two isometric absorption points at arround 360nm and 460nm where the two absorption curves intersect one another. The absorbance values of MetHb at 405nm and 430nm from the baseline are read from the absorption curve of MetHb and represented as b and d, respectively. Similarly, the absorbance values of DeoxyHb at 405nm and 430nm from the baseline are read and represented as a and c, respectively.

A405 (MetHb) and A430 (DeoxyHb) are calculated using the following equations (I) and (II).

$$A405(MetHb)=(b-a)\times Met/100+a \qquad (I)$$

$$A430(DeoxyHbV)=(c-d)\times Met/100+c \qquad (II)$$

wherein Met is the level (%) of methemoglobin that is calculated by the following equation (III).

$$Met(\%)=\{100(a-Qc)\}/\{a-b-Q(c-d)\} \qquad (III)$$

wherein Q=A405(MetHb)/A430(DeoxyHb)

4. Measurement of Met(%) of samples

To a necked photometric cuvette containing 5mL of PBS is added a sample solution of Hb to a Hb concentration of 5g/dL. After bubbling with nitrogen gas for 15 minutes, an absorption curve over a wavelength range from 300nm to 500nm is recorded. Then a baseline connecting two isoabsorbance points at around 360nm and 460nm is drawn and the absorbance values at 405nm and 430nm from the baseline are read. Then Met(%) is calculated using the equations (I),(II) and (III) in the same manner as in Section 3.

Part II. Stability of test samples

Step 1. Preparation of liposome dispersion

To a solution of hemoglobin (Hb) having a Hb concentration of 40g/dL were added an amount of a solution of pyridoxal-5'-phosphate (PLR) in aqueous NaOH and an amount of homocysteine (Hcy) to make the final concentrations of Hb, PLP and Hcy at 5.9mmol/L, 17.3 mmol/L and 5 mmol/L, respectively. The mixture was then adjust to a pH at 7.4, stirred overnight at 4°C, and filtered through a 0.22$\mu$m filter. The resulting solution was repeatedly degassed and carbonylated with CO three times to obtain a solution of carbonylhemoglobin (COHb).

Separately, a mixture of dipalmitoylphosphatidylcholine (DPPC), cholesterol and 1.5-dipalmitoyl-L-glutamate-N-succinic acid in molar ratio of 10/10/2 was dissolved in benzene to a concentration of 20g/L.

To the above benzene solution was added a polyethylene glycol (PEG)-distearyl glutarate ester conjugate (PEG-DSGE) dissolved in physiological saline at a concentration of 1g/L to a ratio of PE-DSGE relative the total lipid of 0.3 mole%. The mixture was then thoroughly mixed and lyophilized to obtain a dry powder of lipid mixture.

The resulting dry powder was added to the COHb solution prepared as above in portions, allowed to hydrate at 4°C, and vigorously stirred overnight. The resulting dispersion was extruded using an extruder (available from Lipexbiomembrances, Inc.) to adjust the average particle size of the liposome to about 220nm. A dispersion of liposome encapsulated hemoglobin having a hemoglobin concentration of about 10g/dL and a lipid concentration of 6g/dL was obtained.

Step 2. Preparation of liposome dispersion containing L-Cys

An aliquot of the above liposome dispersion was thoroughly deoxygenized in a 100mL vial by bubbling with nitrogen gas for 5-6 hours. Separately, L-Cys was dissolved in physiological saline to 28mM and deoxygenated similarly. Test solutions containing L-Cys at levels of 5.6mM, 2.8mM, 1.4mM and 0.7mM, respectively were prepared by mixing the deoxygenated liposome dispersion and the deoxygenated L-Cys solution at ratios in mL of 40:10, 45:5, 47.5:2.5 and 48.75:1.25, respectively. The total volume of the test solution was 50mL.

Step 3. Preparation of liposome dispersion containing sodium hydrogen sulfite

A 40mL aliquot of the deoxygenated liposome dispersion prepared in Step 1 was mixed with 10mL of a deoxygenated solution of sodium hydrogen sulfite in physiological saline having a molar concentration of 50mM to give a test solution containing 5mM of sodium hydrogen sulfite.

Step 4. Preparation of liposome dispersion containing ascorbic acid

Analogous to Step 3, a 40mL aliquot of the deoxygenated liposome dispersion prepared in Step 1 was mixed with 10mL of 50mM solution of ascorbic acid in physiological saline to give a test solution containing 5mM of ascorbic aicd.

Step 5. Preparation of liposome dispersion free from L-Cys

A 45mL aliquot of the deoxygenated liposome dispersion prepared in Step 1 was mixed with 5mL of physiological saline to give a solution free from any reducing agent.

Example 1. Stability of liposome dispersion containing L-Cys in glass vial

A 10mL glass vial (24mm body diameter, 40mm height and 12.5mm mouth opening diameter) was filled with a sample solution prepared in Step 2 containing L-Cys at 0.7mM, 1.4mM or 2.8mM and sealingly closed with a rubber stopper and an aluminum cap. The filling operation was carried out in a atmosphere having an oxygen concentration less than 0.1%. Each vial was stored at 25°C for 6 months. The stability of hemoglobin encapsulated in the liposome was evaluated at days 2, 7, 28 and 6th month in terms of percent methemoglobin (%Met) determined by the method as described above. The results are shown in Table 1 below.

Table 1. %Met of liposome-encapsulated hemoglobin in a glass vial.

| L-Cys Conc. | % Met | | | |
|---|---|---|---|---|
| | day 2 | day 7 | day 28 | 6th month |
| 0.7 mM | -0.2 | -0.1 | 0.3 | 0.2 |
| 1.4mM | -0.7 | 0.1 | 0.1 | -1.6 |
| 2.8 mM | -1.0 | -1.0 | -0.9 | -1.8 |

As demonstrated in Table 1, %Met remained essentially the same upon storage at 25°C up to 6 months.

Example 2. Stability of liposome dispersion in a plastic bag packaged under oxygen-deficient conditions (O$_2$ < 0.3%)

50mL of the sample liposome dispersion containing 2.8mM of L-Cys prepared in Part II was packaged and sealed in a rectangular (8x10cm) bag made of polypropylene (HFB Sheet, Nipro Corporation) in an atmosphere having an oxygen concentration less than 0.3%. The packaged bag was further packaged in an oxygen-impermeable outer envolope (TECHBARRIER™, Mitsubishi Plastics, Inc.) together with an oxygen-absorbing agent (AGELESS™, Mitsubishi Gas Chemical Co., Ltd.). The resulting product was stored at a temperature of 40°C and a relative humidity of 75% or at a temperature of 25°C and a relative humidity of 60%. The level of methemoglobin (Met%) was determined with time in duplicate(n=2). The results are shown in Table 2 below.

Table 2. %Met of liposome-encapsulated hemoglobin in plastic bag packaged at O$_2$ < 0.3%

| Storage conditions | Time | | | | | |
|---|---|---|---|---|---|---|
| | 2 days | 14davs | 28davs | 3mon. | 6mon. | 12mon. |
| Temp.40°C | 1.0 | 1.1 | -2.7 | - | - | - |
| RH75% | 0.9 | 0.7 | -2.7 | - | - | - |
| Temp.25°C | 2.1 | 1.4 | -0.1 | -1.6 | 2.0 | -0.5 |
| RH 60% | 0.0 | 2.8 | 0.2 | -1.8 | -0.1 | -0.3 |

As demonstrated in Table 2 %Met of the sample liposome dispersion in the dual package was not elevated upon storage for 1 month at 40°C and at 75% RH, or for 12 months at 25°C and at 60% RH.

Example 3. Stability of liposome dispersion in a plastic bag packaged under oxygen-deficient conditions (O$_2$ < 3%)

Example 2 was repeated except that the packaging operation was carried out in an atmosphere having an oxygen concentration of 3%. The results are shown in Table 3 below.

Table 3. %Met of lopisome-encapsulated hemoglobin in plastic bag packaged at O$_2$ < 3%

| Storage conditions | Time | | | | | |
|---|---|---|---|---|---|---|
| | 2days | 14days | 28days | 2mon. | 6mon. | 12mon. |
| Temp.40°C | 3.1 | 2.3 | -0.2 | - | - | - |
| RH75% | 1.2 | 1.2 | -0.1 | - | - | - |
| Temp.25°C | 2.7 | 0.3 | -1.0 | -0.7 | -1.5 | -0.4 |
| RH 60% | 2.8 | -1.2 | -2.9 | -1.6 | 1.6 | 0.0 |

As demonstrated in Table 3, %Met of the sample liposome dispersion in the dual package was not elevated upon storage for 1 month at 40°C and at 75% RH, or for 12 months at 25°C and at 60% RH.

Example 4. Stability of liposome dispersion in a plastic bag packaged in the atmosphere (O$_2$=21%)

Example 2 was repeated except that the packaging operation was carried out under the atmospheric conditions (O$_2$=21%). The results are shown in Table 4 below.

Table 4. %Met of liposome-encapsulated hemoglobin in plastic bag packaged at O$_2$=21%

| Storage conditions | Time | | |
|---|---|---|---|
| | Initial | 14davs | 28davs |
| Temp.25°C | 6.4 | 6.0 | 3.6 |

(continued)

| Storage conditions | Time | | |
|---|---|---|---|
| | Initial | 14davs | 28davs |
| RH 60% | 7.0 | 7.7 | 7.2 |

As demonstrated in Table 4, %Met of the sample liposome dispersion in the dual package was not elevated upon storage for 28 days at 25°C and at 60% RH.

Comparative Example 1. Stability of liposome dispersion in a glass vial when L-Cys is not added.

Example 1 was repeated except that the liposome dispersion free from L-Cys prepared in Step 5 was used. The packaging operation was carried out under oxygen-deficient conditions ($O_2 < 0.1\%$). The results are shown in Table 5 below.
Table 5. %Met of liposome-encapsulated hemoglobin free from L-Cys in glass vial

| Storage conditions | Time | | | |
|---|---|---|---|---|
| | 2days | 7days | 28days | 6months |
| Temp.25°C RH 60% | 13.9 | 13.7 | 14.4 | 16.9 |

As demonstrated in Table 5, %Met of the liposome dispersion free from L-Cys in the glass vial was significantly elevated with time upon storage at 25°C and at 60% RH.

Comparative Example 2. Stability of liposome dispersion in a plastic bag when L-Cys is not added.

Example 2 was repeated except that the liposome dispersion free from L-Cys prepared in Step 5 was used and the packaging operation was carried out under the atmospheric conditions ($O_2=21\%$). The results are shown in Table 6 below.
Table 6. %Met of liposome-encapsulated hemoglobin free from L-Cys in plastic bag.

| Storage conditions | Time | | |
|---|---|---|---|
| | Initial | 14days | 28davs |
| Temp.25°C RH 60% | 7.1 | 35.0 | 34.7 |

As demonstrated in Table 6, %Met of the sample liposome dispersion free of L-Cys in the dual package was significantly elevated with time upon storage at 25°C and at 60% RH.
Examples 5, 6, 7 and Comparative Example 3. Stability of liposome dispersion containing L-Cys, NaHSO$_3$ or ascorbic acid compared with the dispersion free from L-Cys in glass vial.
Analogous to Example 1, a glass vial (24mm body diameter, 40mm height, 12.5mm mouth opening diameter) was filled with 10mL of the liposome dispersion containing 5.6mM of L-Cys (Example 5), 5mM of NaHSO$_3$ (Example 6), 5mM of ascorbic acid (Example 7), or not containing L-Cys (Comp. Example 3) prepared in Steps 2-5 and sealingly closed with a rubber stopper and an aluminum cap. The filling operation was carried out an oxygen-deficient atmosphere having an oxygen concentration less than 1% or less than 3%. The stability of liposome dispersion packaged at an oxygen concentration less than 1% or less than 3% are shown in Table 7 and Table 8, respectively.
Table 7. %Met of liposome-encapsulated hemoglobin in glass vial upon storage at 40°C ($O_2 < 1\%$).

| Sample | Reducing agent | %Met after 2 days |
|---|---|---|
| Ex. 5 | L-Cvs | -9 |
| Ex. 6 | $NaHSO_3$ | 28 |
| Ex. 7 | Ascorbic acid | 38 |
| Comp.Ex.3 | None | 40 |

Table 8. %Met of liposome-encapsulated hemoglobin in glass vial upon storage at 40°C ($O_2 < 3\%$).

| Sample | Reducing agent | %Met after 2 days |
|---|---|---|
| Ex. 5 | L-Cys | 25 |
| Ex. 6 | $NaHSO_3$ | 55 |
| Ex. 7 | Ascorbic acid | 60 |
| Comp.Ex.3 | None | 80 |

As demonstrated in Tables 7 and 8, the level of Met (%Met) was not significantly elevated in the liposome dispersion containing L-Cys, $NaHSO_3$ or ascorbic acid while the level of Met (%Met) was significantly elevated when any reducing agent was not added.

Industrial Applicability

In one instance of the present disclosure, the oxygen-carrying blood substitute preparation according to the present invention may find use not only as a blood substitute to be transfused to a hypovolemic patient but also other applications. These applications include, for example, a solution for diluting blood before surgery, a replenishing liquid for blood oxygenators and other extracorporeal circuits, a perfusion liquid of living organs for transplatation, an oxygen-supplying liquid to ischemic sites, a preparation for treating chronic anemia, a perfusion solution for liquid ventilation, a sensitizing preparation for treating cancer, and a culture medium for culturing tissue regenerative cells. The preparation may also find use for transfusion to patients having a rare blood type or patients who reject blood transfusion for regional reasons. Furthermore, the preparation of the present invention may also be useful in the field of veterinary medicine.

**Claims**

1.   Oxygen-carrying blood substitute preparation comprising an oxygen-carrying substance capable of reversibly binding and releasing oxygen in vivo, selected from hemoglobin, an intra- or intermolecularly crosslinked hemoglobin, a hemoglobin polymer a polyethylene glycol-modified hemoglobin polymer, a liposome encapsulated hemoglobin, a conjugate of porphyrin-metal complex and albumin, and a conjugate of porphyrin-metal complex and polyethylene glycole-modified albumin, and L-cysteine in a concentration ranging from 0.5 to 10 mM, effective for preventing at least partially irreversibte oxydation of said oxygen-canying substance.

2.   Oxygen-carrying blood substitute preparation according to claim 1, wherein said oxygen-carrying substance is a liposome encapsulated hemoglobin.

3.   Dual package comprising an inner bag filled with the oxygen-carrying blood substitute preparation of claim 1 or 2, and an outer envelope receiving said inner bag and an oxygen-absorbing packet, said inner bag and said outer envelope being made of a plastic material which is essentially oxygen-impermeable.

**Patentansprüche**

1.   Sauerstoff-transportierendes Blutersatz-Präparat, umfassend eine Sauerstoff-transportierende Substanz, die in der Lage ist, Sauerstoff in vivo reversibel zu binden und freizusetzen, ausgewählt unter Hämoglobin, einem intra- oder intermolekular vernetzten Hämoglobin, einem Hämoglobin-Polymer, einem Polyethylenglykolmodifizierten Hämoglobin-Polymer, einem Liposomen-verkapselten Hämoglobin, einem Konjugat von Porphyrin-Metall-Komplex und Albumin, und einem Konjugat von Porphyrin-Metall-Komplex und Polyethylenglykol-modifiziertem Albumin, und umfassend L-Cystein in einer Konzentration von 0,5 bis 10 mM, das wenigstens teilweise eine irreversible Oxidation der Sauerstoff-transportierenden Substanz verhindert.

2. Sauerstoff-transportierendes Blutersatz-Präparat nach Anspruch 1, wobei die Sauerstoff-transportierende Substanz ein Liposomen-verkapseltes Hämoglobin ist.

3. Dualpackung, umfassend einen inneren Beutel, der mit dem Sauerstoff-transportierenden Blutersatz-Präparat nach Anspruch 1 oder 2 gefüllt ist, und eine äußere Hülle, die den inneren Beutel und ein Sauerstoff absorbierendes Päckchen aufnimmt, wobei der innere Beutel und die äußere Hülle aus einem Plastikmaterial hergestellt sind, das im Wesentlichen Sauerstoff-undurchlässig ist.

**Revendications**

1. Préparation de substitut de sang porteuse d'oxygène, dite aussi transporteuse d'oxygène, comprenant une substance porteuse d'oxygène susceptible de fixer et de libérer de l'oxygène in vivo de façon réversible, sélectionnée parmi l'hémoglobine, une hémoglobine à réticulation intra-ou intermoléculaire, un polymère d'hémoglobine, un polymère d'hémoglobine modifié polyéthylène glycol, une hémoglobine enrobée de liposome, un conjugué d'un complexe porphyrino-métallique et d'albumine, et un conjugué d'un complexe porphyrino-métallique et d'albumine modifiée polyéthylène glycol, et de la L-cystéine à une concentration comprise dans une plage de 0,5 à 10 mM, efficace pour empêcher au moins partiellement une oxydation irréversible de ladite substance porteuse d'oxygène.

2. Préparation de substitut de sang porteuse d'oxygène selon la revendication 1, dans laquelle ladite substance porteuse d'oxygène est une hémoglobine enrobée de liposome.

3. Emballage double comprenant une poche interne emplie de la préparation de substitut de sang porteuse d'oxygène selon la revendication 1 ou 2, et une enveloppe externe recevant ladite poche interne et un sachet d'absorbeur d'oxygène, ladite poche interne et ladite enveloppe externe étant fabriquées en une matière plastique, qui est essentiellement imperméable à l'oxygène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2709419 B
- JP 3466516 B

- WO 9300101 A

### Non-patent literature cited in the description

- Liposome Technology. 1993, vol. 1, 41
- **Tsuchida, E. et al.** *Bioconjugate Chemistry,* 1997, vol. 8, 534-538

- **E. Tsuchida et al.** *Bioconjugate Chemistry,* 1997, vol. 8, 534-538